# EUROPEAN PATENT APPLICATION

(11) **EP 3 951 372 A1**
(43) Date of publication of application: **09.02.2022**
(21) Application number: 19923152.3
(22) Date of filing: 01.04.2019
(51) Int. Cl.: G01N 27/00

(54) **MACHINE-LEARNING PROGRAM, METHOD, AND APPARATUS FOR MEASURING, BY PORE ELECTRIC RESISTANCE METHOD, TRANSIENT CHANGE IN ION CURRENT ASSOCIATED WITH PASSAGE OF TO-BE-MEASURED PARTICLES THROUGH PORES AND FOR ANALYZING PULSE WAVEFORM OF SAID TRANSIENT CHANGE**

(71) Applicant: Aipore Inc., Tokyo, 150-8512 (JP)
(72) Inventor: NAONO, Norihiko, Tokyo 150-8512 (JP)
(74) Representative: Mewburn Ellis LLP
(86) International application number: PCT/JP2019/014544
(87) International publication number: WO 2020/202446

(57) **Abstract**

An apparatus using a feature value extracted from a pulse waveform representing a transient change in ion current flowing between electrodes when a particle passes through a pore, as teacher data and data subject to analysis for machine learning. The apparatus includes a machine-learning program, a searcher, a host attribute table, and a feature value table, a host attribute table is searched using first host attribute information as a search key to extract a first host ID and a second host ID associated with the first host attribute information, a feature value table is searched using a first host ID as a search key to extract a first teacher feature value group obtained from first known particles of a first type, a feature value table is searched using a second host ID as a search key to extract a second teacher feature value group obtained from second known particles of the first type, learning is performed using the first teacher feature value group and the second teacher feature value group as teacher data and first particle type information representing the first type as a teacher label to calculate machine learning optimization parameters, and the machine learning optimization parameters with an input value that is a feature value group subject to analysis obtained from an unknown particle with a first host attribute are used to discriminate whether or not the unknown particle is of the first type.

## Description

### Technical Field

The present invention relates to a machine-learning program, a method, and an apparatus for measuring, by the pore electric resistance method, a transient change in ion current caused when target particles pass through pores, and for analyzing a pulse waveform of said transient change.

### Background Art

As a means for measuring the charge of micron to submicron-size particles such as viruses and bacteria, it has been proposed to measure the transient change of an ion current caused when the target particles in an electrolytic solution pass through pores (Patent Literature 1). Hereinafter, the method of measuring a transient change of the ion current caused when particles pass through pores will be referred to as the pore electric resistance method. In recent years, forming pores using a silicon thin film and reducing the thickness of the pores to about 50 nm has made it possible to measure not only the volume but also information such as the fine structure and surface charge of the target particles (Patent Literature 2). Also, a technique has been proposed in which the transient change of the ion current and the information processing by machine learning are combined to accurately identify the type of the target particles (Patent Literature 3).

The conventional pore electric resistance methods described in these patent literatures use a sensor in which two chambers are connected through pores. Filling the two chambers and pores with an electrolytic solution containing particles and applying a voltage between the two electrodes in contact with the electrolytic solution in each chamber cause an ion current flow. The charged particles move by electrophoresis or the like and pass through the pores. During the passage, the electric resistance between the two electrodes changes transiently. The transient current change is extracted as a pulse waveform, and its waveform information is analyzed by a machine-learning program to identify the type of particles that have passed through the pores.

Such machine learning process uses supervised learning, which includes two steps: the first step of learning and the second step of identification.

The first learning step is performed in the following manner, for example. First, sample particles of a known type are introduced into an electrolytic solution, and one waveform is obtained each time one of these particles passes through the pores. In particle measurement of particles, such as viruses and bacteria, since a large number of particles are contained in the electrolytic solution, a large number of waveforms are usually obtained in one measurement. Therefore, in a machine-learning program in the pore electric resistance method, the teacher feature values extracted from a large number of pieces of waveform information obtained in this way are used as teacher data, and the type of the sample is used as the correct answer label for all the pieces of waveform information, and the optimization parameters of the machine learning model are calculated so that the error function is minimized.

The second identification step is performed in the following manner, for example. A sample containing unknown particles is introduced into an electrolytic solution to obtain the pulse of the unknown particles. Each time one of these unknown particles passes through the pores, one waveform is obtained. The machine-learning program gives the feature values subject to analysis extracted from each of the waveforms obtained here to the machine learning model having the optimization parameters calculated in the first step, so that the probability that each particle that has passed through the pore is of the same type as the particle as a teacher label during learning is calculated. The combination of the pore electric resistance method and machine learning makes it possible to identify each type of particle subject to analysis in this way.

A conventionally disclosed technique (Non Patent Literature 1) has evaluated the accuracy of identification by the following cross-validation process.

For example, a virus strain known to be of type X is propagated by culturing, the group of X-type virus particles is introduced into an electrolytic solution, and a group of pulses caused when X-type particles pass through the pores is then obtained by the pore electric resistance method. Similarly, for example, a virus strain known to be type Y is propagated by culturing, the group of Y-type virus particles is introduced into an electrolytic solution, and a group of pulses caused when Y-type particles pass through the pores is then obtained by the pore electric resistance method.

Next, a part of the X-type particle pulse group and a part of the Y-type particle pulse group are classified as teacher data and the rest as test data. First, the teacher label "X" is assigned to the X-type particle pulse group that was classified as teacher data, and the teacher label "Y" is assigned to the Y-type particle pulse group that was classified as teacher data. They are input to a machine learning model to calculate machine learning optimized parameters.

Next, each of the pulses classified as the remaining test data is input to a machine learning model having the parameters optimized by the teacher data in order to perform verification. Verification is performed multiple times and the average of correct answer rates is determined while changing the classification of the obtained pulse groups into teacher data and test data; thus, a correct answer rate approximate to the actual correct answer rate of the machine learning model can be determined without using a lot of measurement data.

### Citation List

### Patent Literature

Patent Literature 1. Japanese Translation of PCT International Application Publication No. 2014-521962
Patent Literature 2. Japanese Patent No.5866652
Patent Literature 3. Japanese Patent Laid-Open No.2017-120257

### Non Patent Literature

Non Patent Literature 1. ARIMA et al, SCIENTIFIC REPORTS, (2018)8:16305

### Summary of Invention

### Technical Problem

However, even if a favorable correct answer rate is obtained by any of these conventional methods, the learning model would not be guaranteed to have practical value for the following two reasons.

The first reason is that the pulse used for learning and the pulse used for verification are pulse points obtained by one measurement. In the identification of micron- to nano-sized particles such as real viruses and bacteria, learning and identification cannot be made with the pulse group obtained by one measurement. The measurement for identification is performed because the correct type of particles is unclear, and the measurement of particles with a correct answer label that can be learned does not require identification in the first place. Practically, the pulse measurement for learning and the pulse measurement for identification must be different.

The second reason is that the pulse used in learning and the pulse used in verification were generated in the same environment. This cannot happen in practical particle identification either. For example, the shape and surface charge condition vary between viruses obtained by culturing using a developing chicken egg and viruses collected from the human body viruses even if these viruses are of the same type. Moreover, the characteristics may highly possibly vary among viruses of the same type depending on the host. For this reason, practically, viruses of the same type generated in different environments must be identified as of the same type, and viruses of the same type with different hosts must be identified as of the same type.

The conventional techniques proposed above have not implemented a method that enables such practical identification.

### Solution to Problem

The present invention, which has been made in view of such a situation, can provide the following modes/aspects applicable to clinical examination using pulses by the pore electric resistance method in an embodiment of the present invention.

An apparatus for utilizing a structure in which two chambers to be filled with an electrolytic solution containing particles are connected through a pore that a particle can pass through, the two chambers each using a sensor having electrodes to be in contact with the electrolytic solution,
wherein a voltage is applied between the electrodes of the sensor, and a feature value extracted from a pulse waveform representing a transient change in ion current flowing between the electrodes when a particle passes through the pore is used as teacher data and data subject to analysis, thereby performing machine learning,
wherein the apparatus includes storage means,
wherein the storage means includes:
   a machine-learning program;
   a searcher;
   a host attribute table that stores host attribute information on a particle in association with a host ID used to identify the host of the particle; and
   a feature value table that stores a feature value group extracted from a pulse waveform output from the sensor, and particle type information indicating a type of the particle in association with the host ID,
wherein the searcher is configured to search the host attribute table using first host attribute information as a search key, and extract a first host ID and a second host ID associated with the first host attribute information,
wherein the searcher is configured to search the feature value table using the first host ID as a search key and extract a first teacher feature value group obtained from first known particles of a first type, and search the feature value table using the second host ID as a search key and extract a second teacher feature value group obtained from second known particles of the first type,
wherein the machine-learning program is configured to learn using the first teacher feature value group and the second teacher feature value group collectively as teacher data, and first particle type information representing the first type as a teacher label to calculate machine learning optimization parameters, and
wherein the machine-learning program is configured to use the machine learning optimization parameters with an input value that is a feature value group subject to analysis obtained from an unknown particle having the first host attribute information to discriminate whether or not the unknown particle is of the first type.

A machine-learning program, configured to carry out the steps of:
connecting a sensor, wherein two chambers to be filled with an electrolytic solution containing known particles are connected through a pore that known particles can pass through, the two chambers each being connected to the sensor having electrodes to be in contact with the electrolytic solution;
applying a voltage between the electrodes of the sensor to obtain a transient change in ion current flowing between the electrodes when the known particle passes through the pore as a teacher waveform, extracting a teacher feature value from the teacher waveform, and learning the teacher feature value as learning data and the type of the known particle as teacher data to calculate machine learning optimization parameters;
applying a voltage between the electrodes of the sensor to obtain a transient change in ion current flowing between the electrodes when an unknown particle passes through the pore as a waveform subject to analysis, and identifying the type of the unknown particle by using a feature value subject to analysis extracted from the waveform subject to analysis;
calculating a machine learning optimization parameter by using a first teacher feature value obtained from first known particles from a first host and of a first type, and a second teacher feature value obtained from second known particles from a second host and of the first type are used as learning data, wherein the first teacher feature value and the second teacher feature value are collectively used as teacher data for learning to calculate the machine learning optimization parameter; and
inputting an input value that is a first feature value subject to analysis obtained from a first unknown particle from the third host, and using the machine learning optimization parameter to discriminate whether or not the first unknown particle is of the first type.

A machine-learning program, configured to carry out the steps of:
connecting a sensor, wherein two chambers to be filled with an electrolytic solution containing known particles are connected through a pore that known particles can pass through, the two chambers each being connected to the sensor having electrodes to be in contact with the electrolytic solution;
applying a voltage between the electrodes of the sensor to obtain a transient change in ion current flowing between the electrodes when the known particle passes through the pore as a teacher waveform, extracting a teacher feature value from the teacher waveform, and learning the teacher feature value as learning data and the type of the known particle as teacher data to calculate a machine learning optimization parameter;
applying a voltage between the electrodes of the sensor to obtain a transient change in ion current flowing between the electrodes when an unknown particle passes through the pore as a waveform subject to analysis, and identifying the type of the unknown particle by using a feature value subject to analysis extracted from the waveform subject to analysis, and the machine learning optimization parameter;
calculating a machine learning optimization parameters by using a pair of a first teacher feature value group obtained from first known particles from a first host with a first host attribute and of a first type and first host attribute information representing the first host attribute, and a pair of a second teacher feature value group obtained from second known particles from a second host with a second host attribute and of the first type and second host attribute information representing the second host attribute are collectively used as teacher data, and first particle type information representing the first type is used as a teacher label; and
inputting input values that are a first feature value group subject to analysis obtained from an unknown particle from a third host with a third host attribute, and third host attribute information representing the third host, and using the machine learning optimization parameter to discriminate whether or not the unknown particle is of the first type.

### Advantageous Effect of Invention

An embodiment of the present invention enables practical identification of particles applicable to clinical examination and the like by using pulse signals by the pore electric resistance method.

### Brief Description of Drawings

[Figure 1] Figure 1 is a schematic view of an example of the configuration of a sensor module according to the pore electric resistance method.
[Figure 2] Figure 2 shows a schematic example of a pulse waveform detected by the sensor module.
[Figure 3] Figure 3 shows an example of a machine learning system or apparatus according to an embodiment of the present invention.
[Figure 4] Figure 4 shows an example of the configuration of the information terminal shown in Figure 3.
[Figure 5] Figure 5 shows an example of the configuration of a server included in the machine learning system shown in Figure 3.
[Figure 6] Figure 6 shows a flow chart of information processing of learning that may be performed in a certain embodiment of the present invention.
[Figure 7] Figure 7 shows an example of a feature value table.
[Figure 8] Figure 8 shows an example of a host attribute table.
[Figure 9] Figure 9 shows an example of information processing related to identification following the information processing shown in Figure 6.
[Figure 10] Figure 10 is a flow chart of an example of processing for calculating machine learning optimization parameters by extracting a feature value set group from a pulse waveform obtained only from known particles derived from a host having the same host attribute information and using only that feature value set group.

### Description of Embodiments

### Configuration

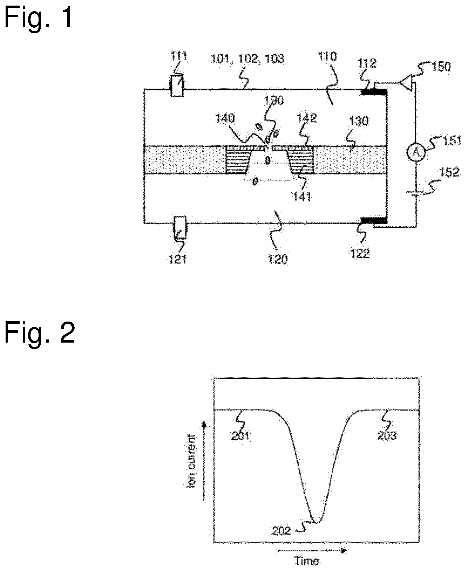

Figure 1 schematically shows an example of the structure of a sensor module according to the pore electric resistance method. Sensor modules 101 to 103 each have electrolytic solution inlets 111 and 121, chambers 110 and 120, a silicon wafer 141, a pore 140 formed by processing a thin film (membrane) 142 deposited on the silicon wafer 141, a partition 130 separating the two chambers, electrodes 112 and 122 provided in the chambers 110 and 120, respectively, a power supply 152 that gives a potential difference between these electrodes, an ammeter 151 that measures the ion current flowing between these electrodes, and an amplifier 150 that amplifies signals. The chamber may be a microchamber. The number assigned to each section shown in Figure 1 refers to a component of a sensor having the same configuration, and does not indicate only a part of a specific sensor module.

In order to identify or discriminate the type of particles to be identified, first, an electrolytic solution containing particles to be identified 190 is introduced from the inlet 111 or 121 and the chambers 110, 120 and the pore 140 are filled with the electrolytic solution. The particles to be identified may be present in both chambers 110 and 120, or may be present in only one of them. The power supply 152 then applies a voltage between the electrodes 112 and 122.
The target particles 190 in the charged chamber move from the chamber 110 to the chamber 120 via the pore 140 by the voltage, for example. At this time, the ion current between the electrodes 112 and 122 is reduced by exhausting the electrolytic solution from the pore 140. After the transient temporal change of the ion current is amplified with the amplifier 150, the ammeter 151 monitors it. Note that Figure 1 merely shows an example of a sensor structure that may be used in an embodiment of the present invention, and may be any sensor that can perform particle identification by machine learning using a transient temporal change of current according to the pore electric resistance method.

Figure 2 shows a schematic example of a pulse waveform detected by the sensor modules 101 to 103. In the example of Figure 2, the horizontal axis represents time and the vertical axis represents ion current flowing between the electrodes 112 and 122. The current value 201 represents a state in which the particles to be identified are separated from the pore 140 in the chamber 110, the current value 202 represents a state in which the ions in the pore 140 are exhausted by the particles when passing through the pore 140 and the current value has dropped accordingly, the current value 203 represents a state in which the particles are separated from the pore 140 in the chamber 120 after passing through the pore 140. In general, there are a large number of particles to be identified in the chamber 110, which means that a large number of pulse waveforms illustrated in Figure 2 are monitored in one measurement.

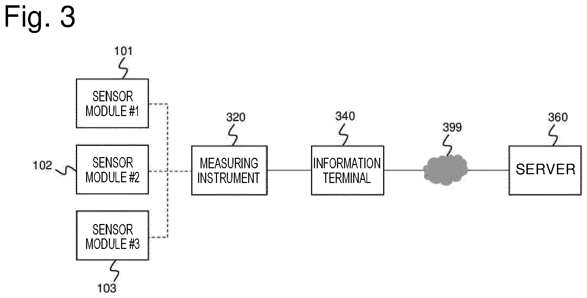

Figure 3 shows an example of a machine learning system or apparatus according to an embodiment of the present invention. The term "apparatus" used herein may be considered as a general term for (collectively including) multiple pieces of hardware included in the system, or may be a term referring to only one of the pieces of hardware. A pulse waveform based on a transient change in the ion current generated in the sensor modules 101 to 103 is sent to a measuring instrument 320 which is used for amplification, current value measurement, and digitization. The measuring instrument 320 may provide the amplifier 150, the ammeter 151, and the power supply 152 shown in Figure 1. The digital-converted pulse waveform is sent to an information terminal 340. In the example shown in Figure 3, the information terminal 340 is connected to a server 360 via a network 399 (in other words, the information terminal 340 may be a client terminal). The network 399 may be a wireless network or a wired network. In some modes, the information terminal 340 may be a mobile terminal connectable to a wireless network. The machine-learning program according to an embodiment of the present invention, which will be described later, may be implemented on either the information terminal 340 or the server 260. Moreover, the present machine-learning program's system configuration for acquiring pulse waveforms is not limited to that shown in Figure 3, and may be any configuration. The dotted lines shown in Figure 3 for explaining the following matters show that the sensor modules 101 to 103 are appropriately reconnected for use and the sensor modules 101 to 103 during use are not always connected to the measuring instrument 320 at the same time. However, another embodiment may use a measuring instrument that can be used with a plurality of sensor modules connected to it at the same time.

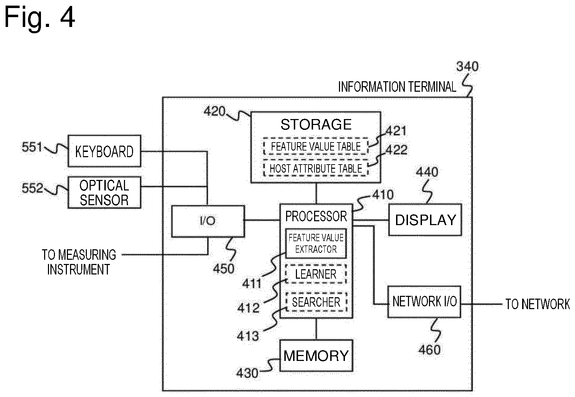

Figure 4 shows an example of the configuration of the information terminal 340 shown in Figure 3. The embodiment shows an example in which the information terminal 340 extracts feature values and another apparatus conducts learning/discrimination. In another embodiment, an apparatus other than the information terminal 340 may perform feature value extraction and learning/discrimination, or the information terminal 340 may perform both feature value extraction and learning/discrimination. The information terminal 340 may include a processor 410, a memory 430, a storage 420, a display 440, an I/O (input/output unit) 450, and a network I/O 460. The processor 410 may be a single-core or multi-core processor, and may physically include a plurality of processors. The I/O 450 receives the digitized pulse waveform information from the measuring instrument 320 (through a keyboard 551, optical sensor 552, or the like) and stores it in the storage 420. The processor 410 can read and use any or all of a feature value extractor (feature value extracting program) 411, a learner 412, and a searcher 413 as pieces of software from the storage 420, the memory 430, or other storage units. In the example shown in Figure 4, the processor 410 reads the feature value extractor 411, may not necessarily read the learner 412 and the searcher 413 enclosed by the dotted lines. The feature value extractor 411 read into the processor 410 extracts a feature value from pulse waveform information. A feature value referred to here is a set of values extracted from a pulse waveform and used as teacher data or identification data for machine learning, expressing the features of the pulse waveform. Hereinafter, a set of feature values generated from a one-pulse waveform will be referred to as a feature value set (feature value group). Since, in general, multiple pieces of pulse waveform information are monitored in one measurement, the feature value extractor 411 often generates as many feature value sets (also referred to as "feature value set groups") as monitored pulses.

In the example shown in Figure 4, the storage 420 does not necessarily hold a table. In another embodiment, the storage 420 may hold a feature value table 421 or host attribute table 422 enclosed by the dotted lines, or both.

In the following description, the feature value (group) extracted from the pulse waveform caused when known particles pass through the pore is referred to as teacher feature value (group), and the feature value (group) extracted when unknown particles pass through the pore is referred to as feature value subject to analysis (group) .

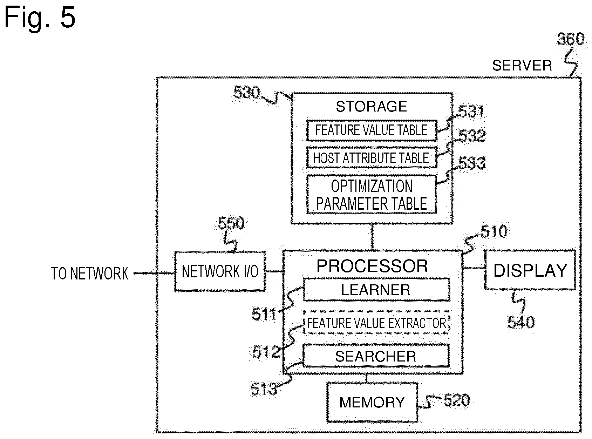

Figure 5 shows an example of the configuration of the server 360 included in the present machine learning system shown in Figure 3. The server 360 may have a processor 510, a memory 520, a storage 530, a display 540, and a network I/O 550. The processor 510 may be a single-core or multi-core processor, and may physically include a plurality of processors. The network I/O 550 receives the host ID, the teacher label indicating the type of known particles, and the teacher feature value set from the information terminal 340 and stores them in the storage 530. The processor 510 may read and use any or all of a learner 511, a feature value extractor 512, and a searcher 513 as pieces of software from the storage 530, the memory 520, or other storage units. In the example shown in Figure 5, the processor 510 is reading the learner 511 and the searcher 513. The learner 511 in the processor 510 calculates machine learning optimization parameters using the teacher label and the teacher feature value set. The term machine learning optimization parameters is a general term for a group of parameters optimized to maximize the probability that the output obtained by inputting a teacher feature value set into a machine learning algorithm matches the true teacher label. The machine learning algorithm used in the present invention may be, for example, a deep learning model, ensemble learning using a decision tree, k-nearest neighbor algorithm, support vector machine, or ensemble learning of a part of these, or is not any of these and may be any mathematical model.

The storage 530 may hold a feature value table 531, a host attribute table 532, and an optimization parameter table 533. The roles of these tables will be explained in detail later.

Here, the host ID is used to identify the place, environment, process, conditions, and the like where the known particles to be the teacher and the unknown particles to be analyzed were generated. For example, when the particles are a virus, it may be used as an ID for identifying the living body where the virus was generated. For example, when an embodiment of the present invention is applied to virus identification in clinical practice, a host ID is assigned to each of the virus particles collected from patient A and the virus particles collected from patient B in order to distinguish them. The host IDs may not be necessarily used only for distinction of the individuals from which the particles are derived, but may also be used to distinguish part or all of the information about the place and environment where the particles were generated, the method and process for generating the particles, and the like.

After calculating the machine learning optimization parameters, the server 360 receives the host ID of unknown particles and the feature value set subject to analysis from the network I/O 550 and stores them in the storage 530. The received feature value set subject to analysis is input to the machine learning algorithm having the machine learning optimization parameters, and the probability that the particle from which the feature value set subject to analysis is derived is the same type of particle as the teacher label is calculated. The process allows the type of the unknown particle to be estimated.

One feature value set subject to analysis is generated for each pulse waveform generated when one particle passes through the pore. Therefore, with the present method, each time a single particle passes through the pore, the type of the particle may be estimated.

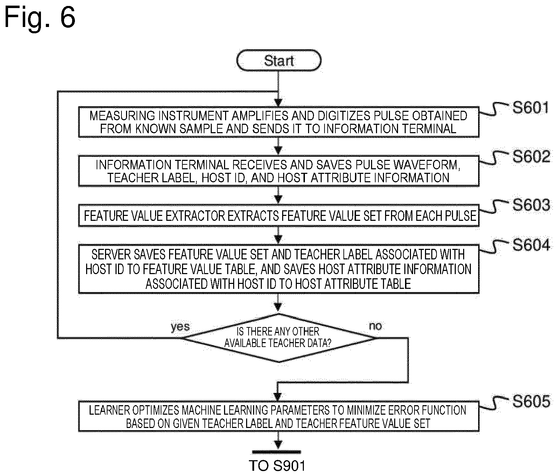

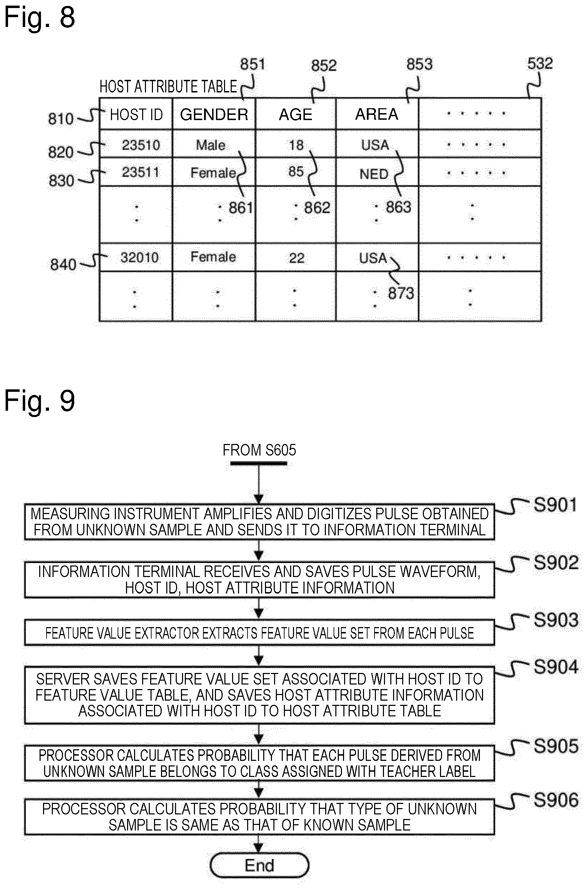

Next, the information processing performed according to an embodiment of the present invention will be explained with reference to the flow charts shown in Figures 6 and 9. In the following description, for easy understanding, the particles are supposed to be viruses, and each host ID is supposed to be an ID for identifying the living body from which the virus was collected. However, these are only examples, and the particles may be any particles that can be measured by the pore electric resistance method, and each host ID may be any information with which the place and environment where the particles were generated, the method, process, conditions, and the like for generating the particles can be identified.

### Learning

Figure 6 shows a flow chart of learning information processing that can be performed according to an embodiment of the present invention. In the following description, for easy understanding, the components shown in Figures 1, 3, 4, and 5 will be cited for description as examples. Needless to say, in another embodiment, other components may be used.

First, an electrolytic solution containing virus particles is generated from a first sample of first known particles collected from a first living body, and is then introduced into the sensor module 101. When a voltage is applied to the electrodes of the sensor module 101, a transient change in the ion current occurs each time the virus particles pass through the pore, and is amplified and digitized by the measuring instrument 320 and sent to the information terminal 340 as a first pulse waveform (Step S601). When the I/O 450 receives the pulse waveform, it is sent to the storage 420. Further, the information terminal 340 acquires information indicating the type of the first known particle, and the first host ID for identifying the first living body and the first host attribute information indicating the attribute of the first host from a keyboard 551, an optical sensor 552, and the like, and these are stored in the storage 420 via the I/O 450 (Step S602). In the example shown in Figure 6, the information terminal 340 acquires these pieces of information from the keyboard or optical sensor, but these may be acquired via the network through the network I/O 460. The information indicating the type of known particle will be referred to as a teacher label below. Since a sample usually contains a large number of particles, a large number of pulse waveforms are obtained by one measurement. For this reason, in Step S601, a plurality of pulse waveforms are stored. These will hereinafter be referred to as a first pulse waveform group. The processor 410 then inputs the first pulse waveform group to the feature value extractor 411, and generates a first feature value set from each of the first pulse waveform groups. As many feature value sets as the first pulse waveforms are generated (Step S603). These sets will hereinafter be referred to as the first feature value set group.

Next, the network I/O 460 sends the first teacher feature value set group, the first teacher label, the first host ID, and the first host attribute information to the server 360 via the network 399. The server 360 stores these pieces of information received at the network I/O 550 in a feature value table 531 and a host attribute table 532 of the storage 530 through the processor 510 (Step S604).

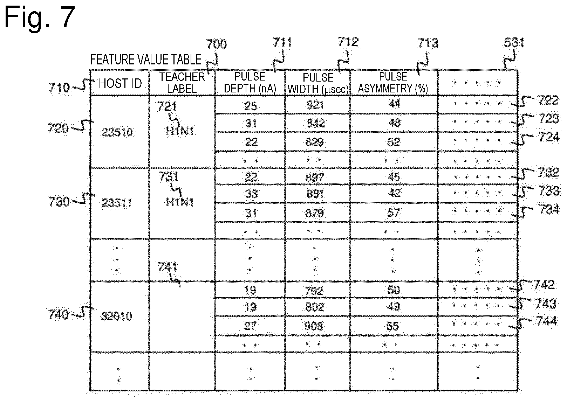

Here, with reference to Figure 7, an example of the aforementioned feature value table is shown. In one example of Figure 7, the teacher label 700, the feature values 711 to 713, and the like are stored in association with the host ID for identifying the host (a note is attached to each column in the heading row 710). In one example shown in Figure 7, a pulse depth 711 in nanoamperes, a pulse width 712 in microseconds, a pulse asymmetry 713 indicated by a percentage, and the like are used as a feature value, and a set of these is stored in association with the corresponding host ID and teacher label. The pulse depth 711 in the example shown in Figure 7 is, for example, the depth from the baseline to the deepest point in the pulse shown in Figure 2, and the pulse asymmetry 713 is, for example, the degree of asymmetry of the pulse shown in Figure 2. The type of feature value that may be used in an embodiment of the present invention is not limited to the example shown in Figure 7, and may be any type of value that represents the feature of a teacher pulse waveform and a pulse waveform subject to analysis.

In one example shown in Figure 7, for example, a teacher feature value set related to one pulse waveform is a set of values stored in the row 722. Figure 7 shows, for example, that a plurality of pulse waveforms are acquired from a sample obtained from a living body represented by the host ID 720 of the first living body and known to contain particles of the teacher label 721. The teacher feature value sets 722, 723, 724, ... acquired from the respective pulse waveforms in association with the host ID 720 and the teacher label 721 are stored.

In the embodiment, the server 360 may additionally receive the attribute information for each host from the information terminal 340 and store it in the host attribute table 532 in association with the corresponding host ID. Figure 8 shows an example of such a host attribute table. In one example shown in Figure 8, gender 851, age 852, area 853, and the like related to the living body that was found to be the host, in association with the host ID are stored in each column (see the heading row 810). The host attribute information that may be used in an embodiment of the present invention is not limited to the example shown in Figure 8, and may be any type of information that represents the host attribute. In another embodiment, the types of tables edited and managed by the information terminal and the server may be different from those in the aforementioned example, and the storage means may be the same or distributed, or physically separated.

Referring back to Figure 6, the sensor then also processes the second sample of the second known particles collected from the second living body. In other words, an electrolytic solution containing virus particles is generated from the second sample of the second known particles collected from the second living body, and is then introduced into the sensor module 102. When a voltage is applied to the electrodes of the sensor module 102, a transient change in the ion current occurs each time the virus particles pass through the pore, and is amplified and digitized by the measuring instrument 320 and sent to the information terminal 340 as a second pulse waveform (Step S601). For example, in the example shown in Figure 3, a sample containing the first known particles is introduced into the sensor module 101, and a sample containing the second known particles is introduced into the sensor module 102, so that the pulse waveforms and the feature values can be extracted. The dotted lines in the example shown in Figure 3 indicate that these sensor modules are not always connected to the measuring instrument 320 at the same time. In another embodiment, these sensor modules may be connected to the measuring instrument 320 at the same time.

For the second known particles also, Steps S601 to S604 are executed in the same manner as for the first known particles. Such processing, the feature value table 531 stores the second teacher feature value set group in association with the second host ID, and the host attribute table 532 stores the second host attribute information in association with the second host ID. In one example of Figure 7, the first host ID is 720, the second host ID is 730, the first teacher label is 721, and the second teacher label is 731. Steps S601 to S604 may be repeated in this way to store the teacher feature value set and the host attribute information acquired from three or more types of known particles.

The processor 510 then inputs the teacher label stored in the feature value table 531 and the stored teacher feature value set together as teacher data to the learner 511. The learner 511 optimizes a number of machine learning parameters of the learner 511 itself so as to minimize the error function. The machine learning parameters optimized here are referred to as machine learning optimization parameters. The processor 510 stores the calculated machine learning optimization parameters in the optimization parameter table 533 (Step S605) .

### Identification

Figure 9 shows an example of information processing related to identification following the information processing shown in Figure 6. In the embodiment, an electrolytic solution containing virus particles is generated from a third sample of unknown particles collected from a third living body that was not used in the processing shown in Figure 6, and is then introduced into the sensor module 103. When a voltage is applied to the electrodes of the sensor module 103, a transient change in the ion current occurs each time the unknown particles pass through the pore, and is amplified and digitized by the measuring instrument 320 and sent to the information terminal 340 as a third pulse waveform group (Step S901). In the example shown in Figure 3, the first sample containing the first known particle is introduced into the sensor module 101, the second sample containing the second known particles is introduced into the sensor module 102, and the third sample containing the unknown particles is introduced into the sensor module 103, so that pulse waveforms can be measured from the respective samples. The dotted lines in the example shown in Figure 3 indicate that these sensor modules are not always connected to the measuring instrument 320 at the same time. In another embodiment, these sensor modules may be connected to the measuring instrument 320 at the same time.

When the I/O 450 receives the third pulse waveform group, it is sent to the storage 420. Further, the information terminal 340 acquires the third host ID for identifying the third living body and the third host attribute information that represents the attribute of the third host from the keyboard 551, the optical sensor 552, and the like, and these are stored in the storage 240 via the I/O 450 (Step S902). Since a sample usually contains a large number of particles, a large number of pulse waveforms are obtained by one measurement. For this reason, a plurality of pulse waveforms are stored. These will hereinafter be referred to as a third pulse waveform group. The processor 410 then inputs the third pulse waveform group to the feature value extractor 411, and generates a feature value set subject to analysis from each of the third pulse waveform groups. As many feature value sets as the pulses are generated from the third pulse waveform group (Step S903). As there are a plurality of feature sets, these will hereinafter be referred to as the third feature value set group. Here, a feature value extracted from the third sample is referred to as a feature value subject to analysis in the sense that it is a feature generated from unknown particles to be analyzed.

Next, the network I/O 460 sends the feature value set group subject to analysis, the third host ID, and the third host attribute information to the server 360 via the network 399. The server 360 stores these pieces of information received at the network I/O 550 in the feature value table 531 and the host attribute table 532 of the storage 530 through the processor 510 (Step S904). In the example shown in Figure 7, the host ID 740 is the host ID of the third host from which the unknown particle was generated. Since unknown particles are used, there is no teacher label for it (the corresponding cell 741 is blank). The feature value set groups 742 to 744 are feature value set groups subject to analysis generated from the third waveform pulse group. In the example shown in Figure 7, the teacher feature value set group and the feature value set group subject to analysis are stored in the same feature value table; alternatively, in another embodiment, the teacher feature value set group and the feature value set group subject to analysis may be stored in different tables.

The processor 510 then inputs the machine learning optimization parameters stored in the optimization parameter table 533 in Step S605 and the feature value set group subject to analysis stored in the feature value table in Step S904 to the learner 511. Then, the learner 511 calculates, for each unknown particle pulse, the probability that the pulse is the same type of pulse as the first known sample (Step S905). In the method, a large number of pulse waveforms of unknown particles are usually monitored in one measurement, and, for each pulse waveform, the probability that the pulse waveform is the same type as the known sample is calculated. The probabilities for the respective pulses are combined to identify whether or not the unknown sample is the same type as the known sample (Step S906). A method of identifying whether or not the unknown sample is the same type as the known sample from the set of probabilities for individual pulse waveforms is, for example, a method of calculating the average of the probabilities for the respective pulses. Alternatively, an embodiment of the present invention may carry out any calculation methods.

As described above, in an embodiment of the present invention, the feature value extractor is located in the information terminal and the learner is located in the server; alternatively, the feature value extractor may be located in the server and feature value extraction in Steps S603 and S903 may be performed in the server 360. The feature value extractor 512 is represented by the dotted line in Figure 5 to show that it may be located in the server. Alternatively, the learner may be located in the information terminal, and the derivation of the machine learning optimization parameters in Step S605 and the identification in Steps S905 to S906 may be performed in the information terminal. The learner 412 in Figure 4 is represented by the dotted line to show that it may be the information terminal. Alternatively, the feature value table and the host attribute table may be held in the information terminal 340. The feature value table 421 and host attribute table 422 in Figure 4 are represented by the dotted lines to show that they may be held in the information terminal 340. Alternatively, either the feature value table or the host attribute table may be held in the server. In the present description, the server and the information terminal may be collectively considered as an "apparatus".

### Highly accurate identification based on host attribute information

In another embodiment, in the learning by the machine-learning program described with reference to Figure 6 and the identification by the machine-learning program described with reference to Figure 9, additional use of host attribute information may yield highly accurate unknown particle identification.

For example, even for the viruses supposed to be of the same type, if there are variants that depend on the attributes of the host, such as the area where the host lives, learning with a machine-learning program using a conventional method causes the learner to learn different features of multiple variants in mixture, which interferes with highly accurate particle identification. Further, for example, even for the particles of the same type in the sense that they act on living cells with the same biological selectivity, in the pore electric resistance method, there may be particles that lead to pulse waveforms having shapes that tend to differ depending on the attributes of the host. In this case also, highly accurate particle identification cannot be achieved for the same reason.

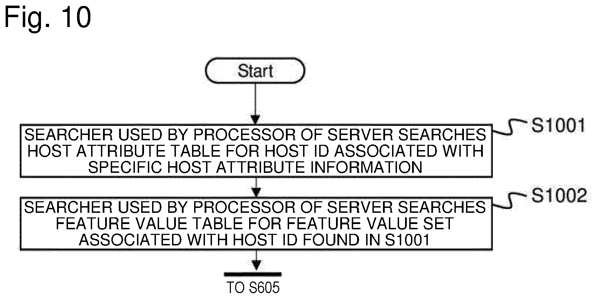

However, in an embodiment of the present invention, unlike the prior art, for example, a feature value set group is extracted from a pulse waveform obtained only from known particles derived from a host having the same host attribute information, and machine learning optimization parameters may be calculated only using the feature value set group. An example of such processing is shown in the flow chart of Figure 10. Illustratively, with reference to Figure 8, the searcher 513 in the processor 510 of the server, for example, searches the column 853 of the host attribute table 532 for "USA" to extract the host ID having host attribute information "USA" (Step S1001). The searcher 513 then extracts the teacher label and the teacher feature value set stored in association with the extracted host ID from the teacher data in the feature value table (Step S1002). Using the teacher label and the teacher feature value set that were extracted here, the learning described in relation to Step S605 is performed, and the machine learning optimization parameters from only the sample having specific host attribute information is calculated. The process can eliminate the need for learning the different features of the pulse waveform of the host-derived particles having the attribute of "USA" and the pulse waveform of other host-derived particles, allowing more highly accurate particle identification. Through learning according to the flow shown in Figure 10, different machine learning optimization parameters are calculated for each host attribute information. Each of these machine learning optimization parameters represents a feature of the particles for each host attribute. The storage 530 of the server may have a host attribute machine learning optimization parameter table that stores the host attribute information and the corresponding machine learning optimization parameters in association with each other.

In the method according to an embodiment of the present invention, as in the example that has been described here, the host attribute table may be searched using one piece of attribute information as a search key, or the host attribute table may be searched using multiple pieces of attribute information as a search key. In this case, learning yields machine learning optimization parameters for each combination of host attributes.

Next, for the identification of unknown particles, prior to the identification flow shown in Figure 9, the machine learning optimization parameters that were learned in the teacher feature value set having the same attributes as the attribute information of the unknown particles to be identified are used for particle identification. To give an example with reference to Figures 8 and 7, first, the host attribute table 532 is searched for the host ID 840 associated with "USA" 873 in the host attribute information on the unknown particles, and the feature value table 531 is searched for the host ID to acquire feature value sets 743 to 744 and the like. The identification processing shown in Figure 9 for each of these feature value sets allows unknown particles having the host ID "32010" to be identified with higher accuracy.

Such processing according to an embodiment of the present invention enables particle identification with high identification accuracy, which is not affected by the difference in host's attributes. Note that the searcher that has been described here may be either on the server or on the information terminal, and the aforementioned processing may be performed either on the server or on the information terminal. For example, the searcher 413 in Figure 4 is represented by the dotted line to show that it may be on the information terminal.

In another embodiment, as the teacher data given to the learner in the learning in Step S605, besides the feature value set stored in the feature value table 531 in Step S604, the host attribute information stored in the host attribute table 532 may be given as a feature value. For example, in the learning in Step S605, in addition to the feature value sets 722, 723, 724 ... stored as teacher data in association with the host ID 720 in the feature value table 531, the host attribute information 863 stored in association with the host ID 820 in the host attribute table 532 may be used as a feature value, and the teacher label 721 may be used as the correct answer for learning.

In still another embodiment, multiple pieces of attribute information stored in the host attribute table 532 may be used as teacher data for learning in Step S605. For example, not only the attribute information 863 but also 862 and 861 may be used as teacher data together with the feature value set associated with the host ID 720. The process allows the machine learning parameters of the machine learning model to undergo optimization including the difference in particles depending on the host. As described above, the machine learning model learned according to an embodiment of the present invention may be used as a machine learning model for particle identification with wider general versatility.

Embodiments of the present invention are able to provide, in addition to the aforementioned method, an apparatus or hardware that can implement the method, a program, and products (e.g., an arbitrary medium, carrier, and module) that store a part or all of the program in a format that is executable by the user.

### Reference Signs List

- 101: Sensor module
- 102: Sensor module
- 103: Sensor module
- 110: Chamber
- 111: Electrolytic solution inlet
- 112: Electrode
- 120: Chamber
- 121: Electrolytic solution inlet
- 122: Electrode
- 130: Partition
- 140: Pore
- 141: Silicon wafer
- 142: Thin film
- 150: Amplifier
- 151: Ammeter
- 152: Power supply
- 190: Target particles
- 201: Current value
- 202: Current value
- 203: Current value
- 320: Measuring instrument
- 340: Information terminal
- 360: Server
- 399: Network
- 410: Processor
- 411: Feature value extractor
- 412: Learner
- 413: Searcher
- 420: Storage
- 421: Feature value table
- 422: Host attribute table
- 430: Memory
- 440: Display
- 450: I/O
- 460: Network I/O
- 510: Processor
- 511: Learner
- 512: Feature value extractor
- 513: Searcher
- 520: Memory
- 530: Storage
- 531: Feature value table
- 532: Host attribute table
- 533: Optimization parameter table
- 540: Display
- 550: Network I/O
- 551: Keyboard
- 552: Optical sensor
- 700: Teacher label
- 710: Heading row
- 711: Column showing feature values for pulse depth
- 712: Column showing feature values for pulse width
- 713: Column showing feature values for pulse asymmetry
- 720: First host ID
- 721: Teacher label
- 722: Teacher feature value set
- 723: Teacher feature value set
- 724: Teacher feature value set
- 730: Second host ID
- 731: Teacher label
- 732: Teacher feature value set
- 733: Teacher feature value set
- 734: Teacher feature value set
- 740: Third host ID
- 741: Blank
- 742: Feature value set subject to analysis
- 743: Feature value set subject to analysis
- 744: Feature value set subject to analysis
- 810: Heading row
- 820: First host ID
- 830: Second host ID
- 840: Third host ID
- 851: Column showing host attribute information (gender)
- 852: Column showing host attribute information (age)
- 853: Column showing host attribute information (area)
- 861: Host attribute information
- 862: Host attribute information
- 863: Host attribute information
- 873: Host attribute information

## Claims

1. An apparatus for utilizing a structure in which two chambers to be filled with an electrolytic solution containing particles are connected through a pore that a particle can pass through, the two chambers each using a sensor having electrodes to be in contact with the electrolytic solution,
wherein a voltage is applied between the electrodes of the sensor, and a feature value extracted from a pulse waveform representing a transient change in ion current flowing between the electrodes when a particle passes through the pore is used as teacher data and data subject to analysis, thereby performing machine learning,
wherein the apparatus includes storage means,
wherein the storage means includes:
a machine-learning program;
a searcher;
a host attribute table that stores host attribute information on a particle in association with a host ID used to identify the host of the particle; and
a feature value table that stores a feature value group extracted from a pulse waveform output from the sensor, and particle type information indicating a type of the particle in association with the host ID,
wherein the searcher is configured to search the host attribute table using first host attribute information as a search key, and extract a first host ID and a second host ID associated with the first host attribute information,
wherein the searcher is configured to search the feature value table using the first host ID as a search key and extract a first teacher feature value group obtained from first known particles of a first type, and search the feature value table using the second host ID as a search key and extract a second teacher feature value group obtained from second known particles of the first type,
wherein the machine-learning program is configured to learn using the first teacher feature value group and the second teacher feature value group collectively as teacher data, and first particle type information representing the first type as a teacher label to calculate machine learning optimization parameters, and
wherein the machine-learning program is configured to use the machine learning optimization parameters with an input value that is a feature value group subject to analysis obtained from an unknown particle having the first host attribute information to discriminate whether or not the unknown particle is of the first type.

2. The apparatus according to claim 1, wherein the apparatus is a server that is connectable to the sensor via a network.

3. A machine-learning program, configured to carry out the steps of:
connecting a sensor, wherein two chambers to be filled with an electrolytic solution containing known particles are connected through a pore that known particles can pass through, the two chambers each being connected to the sensor having electrodes to be in contact with the electrolytic solution;
applying a voltage between the electrodes of the sensor to obtain a transient change in ion current flowing between the electrodes when the known particle passes through the pore as a teacher waveform, extracting a teacher feature value from the teacher waveform, and learning the teacher feature value as learning data and the type of the known particle as teacher data to calculate a machine learning optimization parameter;
applying a voltage between the electrodes of the sensor to obtain a transient change in ion current flowing between the electrodes when an unknown particle passes through the pore as a waveform subject to analysis, and identifying the type of the unknown particle by using a feature value subject to analysis extracted from the waveform subject to analysis, and the machine learning optimization parameter;
obtaining, as learning data, a first teacher feature value from first known particles from a first host and of a first type, and a second teacher feature value obtained from second known particles from a second host and of the first type, and learning the first teacher feature value and the second teacher feature value are collectively used as teacher data to calculate a machine learning optimization parameter, and
inputting an input value that is a first feature value subject to analysis obtained from a first unknown particle from the third host, and using the machine learning optimization parameter to discriminate whether or not the first unknown particle is of the first type.

4. A machine-learning program, configured to carry out the steps of:
connecting a sensor, wherein two chambers to be filled with an electrolytic solution containing known particles are connected through a pore that known particles can pass through, the two chambers each being connected to the sensor having electrodes to be in contact with the electrolytic solution,
applying a voltage between the electrodes of the sensor to obtain a transient change in ion current flowing between the electrodes when the known particle passes through the pore as a teacher waveform, extracting a teacher feature value from the teacher waveform, and learning the teacher feature value as learning data and the type of the known particle as teacher data to calculate a machine learning optimization parameter,
applying a voltage between the electrodes of the sensor to obtain a transient change in ion current flowing between the electrodes when an unknown particle passes through the pore as a waveform subject to analysis, and identifying the type of the unknown particle by using a feature value subject to analysis extracted from the waveform subject to analysis, and the machine learning optimization parameter;
calculating a machine learning optimization parameter by learning a pair of a first teacher feature value group obtained from first known particles from a first host with a first host attribute and of a first type and first host attribute information representing the first host attribute, and a pair of a second teacher feature value group obtained from second known particles from a second host with a second host attribute and of the first type and second host attribute information representing the second host attribute that are collectively used as teacher data, and first particle type information representing the first type which is used as a teacher label; and
inputting input values that are a first feature value group subject to analysis obtained from an unknown particle from a third host with a third host attribute and third host attribute information representing the third host, and using the machine learning optimization parameter to discriminate whether or not the unknown particle is of the first type.

5. The machine-learning program according to claim 3 or 4, wherein the known particle and the unknown particle are viruses.

6. The machine-learning program according to claim 3 or 4, wherein the known particle and the unknown particle are bacteria.

7. The machine-learning program according to claim 3, further configured to carry out the steps of:
having received the teacher waveform and the waveform subject to analysis from the sensor, generating, by an information terminal, the first teacher feature value, the second teacher feature value, and the first feature value subject to analysis;
sending, from the information terminal to a server via a network, the first teacher feature value, the second teacher feature value, and the first feature value subject to analysis; and
executing, by the server, the learning and the discrimination.

8. The machine-learning program according to claim 4, further configured to carry out the steps of:
having received the teacher waveform and the waveform subject to analysis from the sensor, generating, by an information terminal, the first teacher feature value group, the second teacher feature value group, and the first feature value group subject to analysis,
sending, from the information terminal to a server via a network, the first teacher feature value group, the second teacher feature value group, and the first feature value group subject to analysis; and
executing, by the server, the learning and the discrimination.
